# EUROPEAN PATENT SPECIFICATION

(11) **EP 2 014 230 B1**
(45) Date of publication and mention of the grant of the patent: **09.03.2011**
(21) Application number: 07380205.0
(22) Date of filing: 11.07.2007
(51) Int. Cl.: A61B 5/107, G01B 11/02, A01K 29/00

(54) **Device for measuring anatomical parameters in pets**
Vorrichtung zur Messung anatomischer Parameter bei Haustieren
Dispositif de mesure de paramètres anatomiques pour les animaux domestiques

(43) Date of publication of application: 14.01.2009
(73) Proprietor: Verez Fraguela, José Luis, 15404 Ferrol - La Coruña (ES)
(72) Inventor: Verez Fraguela, José Luis, 15404 Ferrol - La Coruña (ES)
(74) Representative: ABG Patentes, S.L.

(56) References cited:
- EP-A- 1 537 778
- JP-A- 10 005 193
- US-A- 4 170 961
- US-A- 5 898 169
- US-A1- 2005 155 246

## Description

### OBJECT AND TECHNICAL FIELD OF THE INVENTION

The present invention relates to a device that has been specially designed to simultaneously measure certain anatomical parameters in pets, e.g. dogs, said parameters being the animal's weigh, height and length.

The object of the invention is to quickly, simply and precisely obtain the value of said parameters in order to verify whether the animal is overweight and whether young animals are developing properly and to diagnose and assess a predisposition to suffer different chronic diseases with or without obesity and to monitor the response to the therapeutic and control processes of the established treatment. The possibility of combining these parameters in a single clinical action makes the task easier, simplifying it by increasing the patient and owners' collaboration.

A dysfunction or imbalance between these parameters is widely known to signify a risk factor that affects the animal's state of health and is a very common factor in chronic diseases such as: disorders of the musculoskeletal system, heart disease, diabetes, hypertension, stroke and some types of cancer. Evidence suggests that this imbalance between height, length and weight involves a disorder resulting from various different factors: genetic, environmental, psychological or family factors, among others, which must be regularly controlled by processes that enable us to obtain the largest amount of information possible in a minimal number of actions, the process making it possible to interrelate the information obtained from the examination.

### BACKGROUND OF THE INVENTION

It is normal to measure said parameters in the case of people, for the aforementioned purposes. The applicant has no knowledge of the use of such parameters for a similar purpose in pets.

Therefore, nothing is known about the existence of devices capable of simultaneously taking such measurements in animals.

European Patent with publication number EP 1452132, belonging to Kasahara Isiokawa, discloses a method for determining weight and impedance and using these values to calculate the body fat percentage and body water percentage in order to assess the animal's state of health, a method wherein the animal's body measurements, i.e. it's length or height, are obviously not used.

US 2005/155246 Al discloses an apparatus for measuring weight, height and/or length, comprising an electronic weighing measuring unit, a laser measuring instrument, and a microcomputer so as to obtain the height based on the distance signal detected by the height detector, the laser measuring instrument having means for regulating its position and the direction of the emitted laser beam.

A similar apparatus is disclosed in JP 10 005193, which comprises a mount board 1 supported on a base 3 via a load cell 2 and a rear material 6 erected on the rear side of the base 4 via a hinge 5. An eaves part 7 extending horizontally is linked with the top part of the rear material 6 and a laser oscillator and a receive 8 are installed downward in a position just above the centre of the mount board 1 of the eaves part 7. The stature is measured by the time difference in receiving the reflection wave of the laser from the laser oscillator, and the maximum value of the measured values is selected, set to the stature measuring value, and displayed on the display part 12.

### DESCRIPTION OF THE INVENTION

The objectives of the invention are achieved by means of a device according to claim 1. The particular embodiments of the device that is the object of the invention are defined in dependent claims 2 to 5.

The device proposed by the invention fills this gap in the prior art, making it possible to simultaneously weigh the animal using the conventional electronic method, and at the same time measure its height and length from its head to its rear end, using a laser measuring instrument for the latter two parameters.

Both weighing and measuring systems are non-invasive and thus bloodless, and they can be linked to each other by computer processing so that, together with the animal's body mass, it is also possible to obtain the parameters either separately or duly interrelated. The data obtained can therefore be used to compare with standard ideal data to be found in the literature for the animal species in question, as well as the breed, sex and other parameters, which enables us to establish a relationship that in turn makes it possible, by comparison with what is considered normal, to detect any anomaly, thus making this another tool with which to monitor a pet's general health.

A first aspect of the invention relates to a device that takes the form of a weighing platform, of a suitable size for the type of animal for which it is to be used, and a support for the laser measuring instrument, which allows said measuring instrument to be moved both vertically and transversally, whilst it can also be rotated to change the direction of the laser beam, so that by means of this multiple mobility of the laser measuring instrument the laser beam can be made to fall on any point of the animal's body in order to make the necessary measurements, e.g. between the ground and its withers, between its head and its tail, etc.

According to a preferred practical embodiment of the invention, the laser measuring instrument is positioned laterally in relation to the weighing platform, i.e. in relation to the animal's body, although said laser measuring instrument can also be situated in an upper position, i.e. over the weighing platform and above the animal.

In any of the cases and according to the invention a sheet of treated glass is situated between the laser measuring instrument and the animal, which allows the laser beam to pass through, causing a refraction effect thereon so that the measurements is taken on said sheet of treated glass at the points where the refracted laser beam is horizontal or vertical, as applicable, and passes through the point to be measured. This is achieved by moving the laser measuring instrument as appropriate until the angle of refraction is at 90° to the plane of the sheet of treated glass.

Logically, the weighing platform is suitably delimited by two side walls or by one wall and by said sheet of treated glass in order to limit the area in which the animal must be situated while the measurements are taken, with a sufficient degree of clearance.

The above-described structure works with a computer program that uses the information supplied by the weighing platform and the laser measuring instrument to perform the necessary calculations and comparisons in order to achieve a diagnosis, in particular the following:
- Calculation of the net weight.
- Calculation of the height.
- Calculation of the length.
- Calculation of the difference between the reference length and the present length.
- Calculation of the difference between the reference height and the present height.
- Calculation of the difference between the reference weight and height and the present weight and height.
- Calculation of the quotient between the weight and the square of the length.
- Capacity to express ideal data and values on the basis of data obtained from the weight measurements.

With the results of these calculations it is possible to carry out the following processes:
- Control of weight, recording Tare Weight and Net Weight.
- Control of length and height.
- Coin access feature.
- Issue of payment receipts, calculating the VAT.
- Issue of data and tables with values and recommendations.
- Detailed data selection for issuing reports.
- Separate detailed reports.
- Detailed report of the calculated values.

It is thus possible to achieve a device that is very simple to use and easy to learn how to manage, with a simple software installation, an automatic installation configuration, restrictive access, independent data tables and a wide range of configuration options.

### DESCRIPTION OF THE DRAWINGS

To complement this description and in order to aid a better understanding of the invention's characteristics, according to a preferred practical embodiment thereof, there is a set of illustrative and non-limiting drawings integral to said description, which are as follows:
- Figure 1: Shows a schematic side elevation view of a device for measuring anatomical parameters in pets, in an embodiment according to the object of the present invention, in which a dog is situated.
- Figure 2: Shows a schematic view similar to that of the previous figure of the same assembly as in said figure, but in a front elevation view.
- Figures 3 and 4: Show similar views to those of figures 1 and 2, but corresponding to a variant of an embodiment of the device in which the laser measuring instrument is situated above the animal, instead of being situated laterally as is the case in the device shown in figures 1 and 2.

### PREFERRED EMBODIMENT OF THE INVENTION

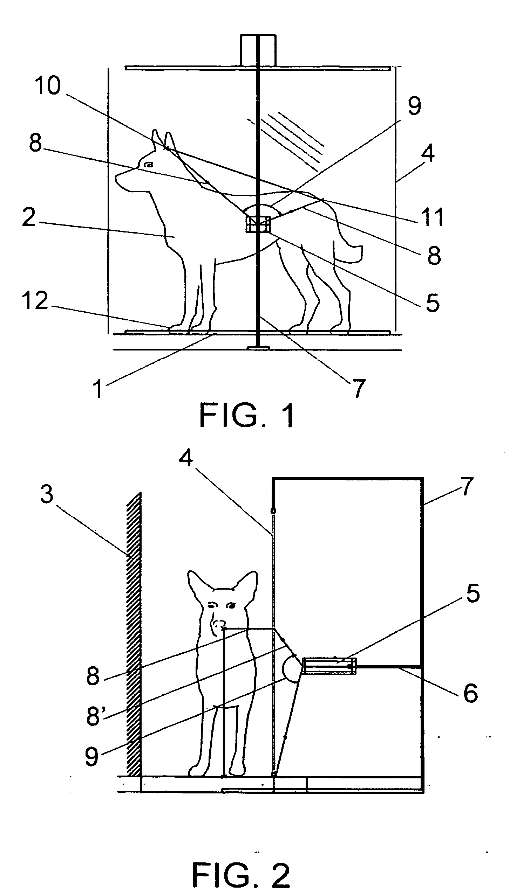

In view of said figures, and particularly figures 1 and 2, it can be seen that the device proposed by the invention consists of an electronic weighing platform (1) of suitable dimensions to fit the dog (2) or animal whose parameters are to be measured, said platform (1) being surrounded in this case by a fixed wall (3) and by the sheet of treated glass (4), as can be seen in figure 2, a laser measuring instrument (5) laterally situated in relation to said sheet of treated glass (4) and opposite the animal (2) capable of moving transversally on a horizontal guide rail (6), it also being possible for said laser measuring instrument to tilt so that the laser beam (8) may be pointed in any direction that is required, as is shown by the arrow (9) in either of figures 1 or 2.

As has been mentioned above, the purpose of the sheet of treated glass (4) is that when the laser beam (8) falls thereon, its path will be changed due to refraction, so that the end section (8') of said laser beam (8) is horizontal and aligned with the points of the animal (2) at which it is to be measured, e.g. at its head (10), its tail (11), or the free end of its legs (12), i.e. at floor level.

Figure 1 shows the laser beams generated by the measuring instrument (5) to determine the length of the dog's body, i.e. the distance between its head and its tail, whilst in figure 2 the same is done to determine its height.

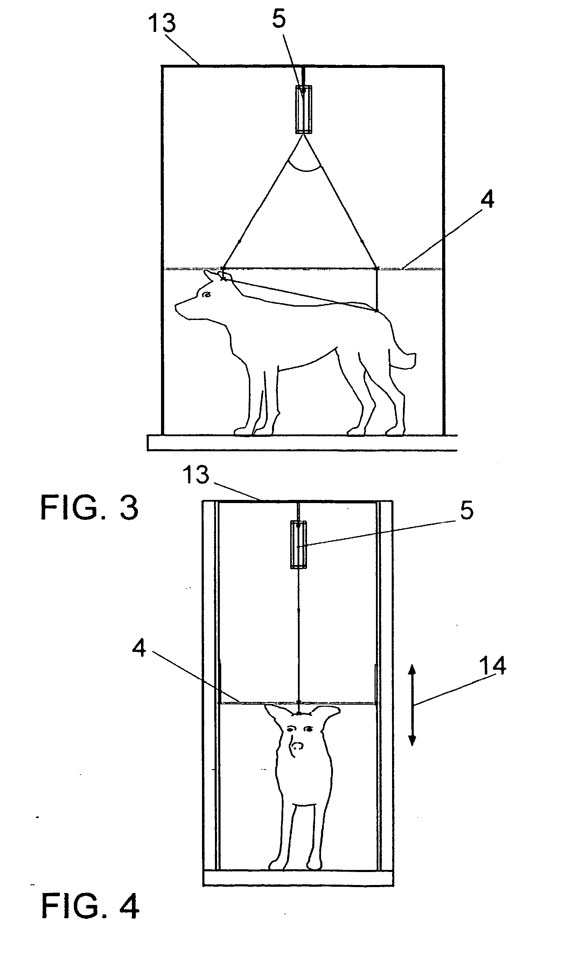

It is also possible, as is shown in figures 3 and 4, for the laser measuring instrument (5) to be situated above the weighing platform (1), i.e. above the animal, in which case said laser measuring instrument can only be moved transversally along a longitudinal guide rail (13), the sheet of treated glass (4) being situated in this case between the animal and the laser measuring instrument, and between the animal and the laser measuring instrument, and in this case it being the sheet of treated glass (4) whose height can be adjusted, as is shown by the arrow (14) in figure 4, rather than the laser measuring instrument's, as in the previous case.

## Claims

1. Device for measuring anatomical parameters in pets, particularly their weight, height and length, comprising:
an electronic weighing platform (1),
at least one laser measuring instrument (5), having means of regulating its position and the direction of the emitted laser beam,
a computer system for processing the data obtained and for processed data output,
**characterised in that**:
between the laser measuring instrument (5) and the area where the animal is situated (2) on the weighing platform (1) there is a sheet of treated glass (4) that the laser beam passes through and which has a refraction effect on the beam such that the refracted section of said laser beam is perpendicular to the plane on which the sheet of treated glass is situated and on which the measurement is taken.

2. Device for measuring anatomical parameters in pets, according to claim 1, **characterised in that** the weighing platform (1) is laterally surrounded by fixed walls (3) or by a fixed wall and the sheet of treated glass (4), delimiting the area where the animal is situated (2) on said weighing platform (1).

3. Device for measuring anatomical parameters in pets, according to the previous claims, **characterised in that** the laser measuring instrument (5) is situated laterally in relation to the weighing platform (1) and therefore also in relation to the animal (2), and it is mounted so that it can move on a horizontal guide rail (6) that in turn can move vertically on a second vertical guide rail (7).

4. Device for measuring anatomical parameters in pets, according to claims 1 to 2, **characterised in that** the laser measuring instrument (5) is situated above the weighing platform (1), i.e. above the animal (2), said laser measuring instrument (5) being movable on a longitudinal and horizontal guide rail (13), whilst the height of the sheet of treated glass (4) can be adjusted, i.e. towards or away from the laser measuring instrument (5).

5. Device for measuring anatomical parameters in pets, according to the previous claims, **characterised in that** the computer system has a means of transmitting the data taken by the weighing platform (1) and the laser measuring instruments (5) to a data processing system, which consists of a computer with a database, and an information output system for the processed data in list form, an information screen, using graphs or the like.

## Patentansprüche

1. Vorrichtung zum Messen anatomischer Parameter bei Haustieren, insbesondere ihres Gewichts, ihrer Höhe und Länge, umfassend:
eine elektronische Wägeplattform (1);
wenigstens ein Lasermessinstrument (5) mit Einrichtungen zum Regulieren seiner Position und der Richtung des ausgestrahlten Laserstrahls;
ein Computersystem zum Verarbeiten der erhaltenen Daten und zur Ausgabe der verarbeiteten Daten;
**dadurch gekennzeichnet, dass**:
es zwischen dem Lasermessinstrument (5) und der Fläche, wo sich das Tier befindet (2), auf der Wägeplattform (1) eine Platte aus behandeltem Glas (4) gibt, durch die der Laserstrahl hindurchtritt und die eine Brechungswirkung auf den Strahl ausübt, so dass der gebrochene Abschnitt des Laserstrahls senkrecht zu der Ebene steht, auf der sich die Platte aus behandeltem Glas befindet und auf der die Messung vorgenommen wird.

2. Vorrichtung zum Messen anatomischer Parameter bei Haustieren gemäß Anspruch 1, **dadurch gekennzeichnet, dass** die Wägeplattform (1) seitlich von fixierten Wänden (3) oder von einer fixierten Wand und der Platte aus behandeltem Glas (4) umgeben ist, wodurch die Fläche abgegrenzt wird, wo sich das Tier auf der Wägeplattform (1) befindet (2).

3. Vorrichtung zum Messen anatomischer Parameter bei Haustieren gemäß den vorstehenden Ansprüchen, **dadurch gekennzeichnet, dass** sich das Lasermessinstrument (5) seitlich von der Wägeplattform (1) und daher auch von dem Tier (2) befindet und so montiert ist, dass sie sich auf einer horizontalen Führungsschiene (6) bewegen kann, welche sich wiederum auf einer zweiten, vertikalen Führungsschiene (7) vertikal bewegen kann.

4. Vorrichtung zum Messen anatomischer Parameter bei Haustieren gemäß Anspruch 1 bis 2, **dadurch gekennzeichnet, dass** sich das Lasermessinstrument (5) oberhalb der Wägeplattform (1), d.h. oberhalb des Tiers (2), befindet, wobei das Lasermessinstrument (5) auf einer longitudinalen und horizontalen Führungsschiene (13) beweglich ist, während die Höhe der Platte aus behandeltem Glas (4) justiert werden kann, d.h. auf das Lasermessinstrument (5) zu oder davon weg.

5. Vorrichtung zum Messen anatomischer Parameter bei Haustieren gemäß den vorstehenden Ansprüchen, **dadurch gekennzeichnet, dass** das Computersystem eine Einrichtung zur Übermittlung der durch die Wägeplattform (1) und die Lasermessinstrumente (5) erhaltenen Daten zu einem Datenverarbeitungssystem, das aus einem Computer mit einer Datenbank besteht, und ein Informationsausgabesystem für die verarbeiteten Daten in Listenform, einen Informationsbildschirm unter Verwendung von Graphiken oder dergleichen aufweist.

## Revendications

1. Dispositif pour mesurer des paramètres anatomiques chez des animaux domestiques, en particulier leurs poids, hauteur et longueur, comportant :
une plate-forme de pesage électronique (1),
au moins un instrument de mesure par laser (5), ayant des moyens pour réguler sa position et la direction du faisceau laser émis,
un système informatique pour traiter les données obtenues et pour la sortie des données traitées,
**caractérisé ce que** :
entre l'instrument de mesure par laser (5) et la zone où se situe l'animal (2) sur la plate-forme de pesage (1), est disposée une feuille de verre traité (4) que le faisceau laser traverse et qui a un effet de réfraction sur le faisceau de telle sorte que la partie réfractée dudit faisceau laser est perpendiculaire au plan sur lequel la feuille de verre traité se situe et sur lequel la mesure est prise.

2. Dispositif pour mesurer des paramètres anatomiques chez des animaux domestiques, selon la revendication 1, **caractérisé en ce que** la plate-forme de pesage (1) est latéralement entourée par des parois fixes (3) ou par une paroi fixe et la feuille de verre traité (4), délimitant la zone où l'animal (2) se situe sur ladite plate-forme de pesage (1).

3. Dispositif pour mesurer des paramètres anatomiques chez des animaux domestiques, selon les revendications précédentes, **caractérisé en ce que** l'instrument de mesure par laser (5) se situe latéralement par rapport à la plate-forme de pesage (1) et donc également par rapport à l'animal (2), et **en ce qu'**il est monté de telle sorte qu'il peut se déplacer sur un rail de guidage horizontal (6) qui peut à son tour se déplacer verticalement sur un second rail de guidage vertical (7).

4. Dispositif pour mesurer des paramètres anatomiques chez des animaux domestiques, selon les revendications 1 à 2, **caractérisé en ce que** l'instrument de mesure par laser (5) se situe au-dessus de la plate-forme de pesage (1), c'est-à-dire au-dessus de l'animal (2), ledit instrument de mesure par laser (5) étant mobile sur un rail de guidage longitudinal et horizontal (13), alors que la hauteur de la feuille de verre traité (4) peut être ajustée, c'est-à-dire en direction de l'instrument de mesure par laser (5) ou en s'éloignant de celui-ci.

5. Dispositif pour mesurer des paramètres anatomiques chez des animaux domestiques, selon les revendications précédentes, **caractérisé en ce que** le système informatique a des moyens pour transmettre les données acquises par la plate-forme de pesage (1) et l'instrument de mesure par laser (5) à un système de traitement de données, lequel est constitué d'un ordinateur avec une base de données, et d'un système de sortie d'informations pour les données traitées sous forme de liste, un écran d'informations en utilisant des graphiques ou des moyens analogues.
